# EUROPÄISCHE PATENTSCHRIFT

(11) **EP 3 538 029 B1**
(45) Veröffentlichungstag und Bekanntmachung des Hinweises auf die Patenterteilung: **10.05.2023**
(21) Anmeldenummer: 17787164.7
(22) Anmeldetag: 19.10.2017
(51) Int. Cl.: A61F 2/28, A61F 2/30

(54) **GELENKIMPLANTAT ZUR GEWEBENEUBILDUNG AM GELENK**
JOINT IMPLANT FOR NEW TISSUE FORMATION AT THE JOINT
IMPLANT ARTICULAIRE POUR LA RÉGÉNÉRATION TISSULAIRE AU NIVEAU DE L'ARTICULATION

(30) Priorität: 12.12.2016 DE 102016124049
(43) Veröffentlichungstag der Anmeldung: 18.09.2019
(73) Patentinhaber: Karl, Christoph, 8274 Tägerwilen (CH)
(72) Erfinder: Karl, Christoph, 8274 Tägerwilen (CH)
(74) Vertreter: Kindermann, Peter
(86) Internationale Anmeldenummer: PCT/EP2017/076691
(87) Internationale Veröffentlichungsnummer: WO 2018/108360

(56) Entgegenhaltungen:
- EP-A1- 1 450 875
- EP-A1- 2 465 549
- WO-A2-2007/020449
- WO-A2-2012/096997
- CN-A- 104 645 419
- US-A1- 2005 221 072
- US-A1- 2008 249 637
- US-A1- 2009 029 077

## Beschreibung

Die vorliegende Erfindung bezieht sich auf ein Gelenkimplantat zur Gewebeneubildung am Gelenk und insbesondere auf ein Gelenkimplantat zur Knorpelneubildung am Knie-, Hüft-, Schulter-, Sprung-, Zehengrund- oder Handgelenk.

Gelenkerkrankungen (Arthrose) gehören zu den zehn häufigsten Erkrankungen weltweit. Ein Totalersatz des erkrankten Gelenks ist hierbei mit hohen Kosten verbunden und wird üblicherweise von den betroffenen Patienten als psychisch belastend empfunden. Revisionen des Gelenkersatzes wirken sich mit weiteren Kosten, Belastungen für den Patienten und häufig Komplikationen aus. Es haben sich daher verschiedene Ansätze zur kurativen Behandlung von Gelenkerkrankungen (Arthrose) und insbesondere zur kurativen Behandlung von Knie- und Hüftgelenks-Arthrose entwickelt.

Die derzeit existierenden Ansätze zur kurativen Behandlung von Gelenkerkrankungen werden nachfolgend erläutert.

Medikamentöse Therapieoptionen: Die medikamentösen Therapieoptionen beschränken sich auf die symptomatische Anwendung von Antiphlogistika und Analgetika sowie die teils intraartikuläre und teils systemische Behandlung mit Hyaluronsäure, Chondroitinsulfat, Interleukin-1 Rezeptorantagonisten und Glucosaminsulfat. Obwohl hierbei gute Ergebnisse bei der Schmerzreduktion nachgewiesen wurden, konnte ein Fortschreiten der Arthrose bislang jedoch damit nicht verhindert werden.

Operative Therapien: Auch operative Therapien wie lokale Knochen- oder Knorpeltransplantation bzw. autologe Chondrozyten-Transplantation oder -Implantation (ACT oder ACI) konnten sich noch nicht durchsetzen, da hierbei jeweils zwei Operationen notwendig sind (Entnahme und Replantation), was eine lange Entlastung bzw. Ruhigstellung des Gelenks während der Rehabilitation bedeutet und sich somit ungünstig auf die Regenerierung auswirkt. Außerdem wird der noch gesunde Knorpel an der Entnahmestelle geschädigt.

Am weitesten verbreitet sind deshalb operative Therapien wie die sogenannte Pridie-Bohrung, die anterograde/retrograde Anbohrung und die Mikrofrakturierung. Bei diesen operativen Therapien erfolgt kein lokaler Knorpelersatz, sondern es werden z.B. mehrere Bohrungen durch die subchondrale Grenzlamelle durchgeführt. Bei der Bohrung nach Pridie und deren Weiterentwicklung *"*Mikrofrakturierung*"* kann im Defektareal ein Einbluten in den Knorpeldefekt erzielt werden, wodurch Fibrozyten, mesenchymale Stammzellen und Chondroblasten aus dem spongiösen Raum in den Knorpeldefekt eingeschwemmt werden. Diese bilden mit Wachstumsfaktoren ein Blutkoagel (*"*super clot*"*) und differenzieren sich zu artikulärem Knorpel. Klinische Studien zeigten Schmerzverringerung und eine gute Gelenkbeweglichkeit. Ein Problem ist jedoch auch hier eine lange Entlastung bzw. Ruhigstellung des Gelenks, wobei sich ein in der Regel minderwertig regenerativer Faserknorpel entwickelt. Dieser ist aufgrund seiner Struktur für die hohen mechanischen Belastungen insbesondere im Kniegelenk häufig nur unzureichend beschaffen und degeneriert schnell, was erneute Eingriffe erforderlich machen kann.

Aus diesem Grund wurden Carbonstifte als Gelenkimplantate zur Gewebeneubildung am Gelenk entwickelt, welche in die Bohrungen eingesetzt werden und zu einem schnellen Überwachsen führen sollen.

Aus der EP 1 450 875 A1 ist ein derartiges Gelenkimplantat zur Gewebeneubildung am Gelenk bekannt, wobei die eingesetzten Stifte aus verdichtetem Kohlenstoff mit einer vorbestimmten Porosität bestehen. Auch bei Verwendung derartiger herkömmlicher Carbonstifte können Fibrozyten und mesenchymale Stammzellen aus dem spongiösen Raum in den Knorpeldefekt eingeschwemmt werden, die ein *"*super clot*"* bilden und sich zu artikulärem Knorpel differenzieren.

Das System hat sich jedoch aufgrund zweier wesentlicher Nachteile noch nicht durchgesetzt. Einerseits ist Kohlenstoff als Werkstoff im Knorpel bei Orthopäden nicht akzeptiert, da Mikroabrieb befürchtet wird. Andererseits ist die Oberfläche nicht auf das Ansiedeln von Stammzellen ausgelegt, was sich auch hierbei in der Entwicklung eines minderwertig regenerativen Faserknorpels zeigt.

Aufgrund dieser Limitierungen konnte sich keine der vorstehend genannten Therapien bisher als *"*Standard of Care*"* durchsetzen.

Aus der Druckschrift WO2007/020449 A2 ist ferner ein implantierbares knorpeliges Reparaturgewebe bekannt, welches eine biokompatible Faserlage und ein biokompatibles poröses Hydrogel aufweist. Durch chemische Funktionalisierung von Faserlage und Hydrogel können die Resorptionsraten eingestellt und insbesondere können hydrophobe Gruppen in hydrophile Ketten umgewandelt werden. Ferner wird das Einbringen von Wachstumsfaktoren in das Reparaturgewebe beschrieben.

Weiterhin offenbart die Druckschrift US2008/0249637 A1 ein teilweise biodegradierbares Implantat zur Reparatur von Knochen oder Knorpeldefekten, wobei eine trabekulare Rahmenstruktur in ein davon verschiedenes Material eingebettet ist.

Schließlich ist aus der Druckschrift EP 2 465 549 A1 ein poröses orthopädisches Implantat sowie ein zugehöriges Herstellungsverfahren bekannt, wobei eine Polymer-Vorform mit einer Vielzahl von Stegen ausgebildet, die Polymer-Vorform pyrolisiert und abschließend die pyrolisierte Vorform mit Metall beschichtet wird.

Ferner ist aus der Druckschrift WO 2012/096997 A2 ein orthpädisches Knorpel-Implantat mit IPN- oder Semi-IPN-Aufbau (Interpenetrating Polymer Networks) bekannt, welches mit thermoplastischen oder thermisch einstellbaren Polymeren lokale Gelenkfehlstellen auffüllen kann.

Weiterhin ist aus der Druckschrift US 2009/029077 A1 eine arzneimittelabgebende implantierbare Vorrichtung mit zumindest einer porösen Schicht bekannt, wobei die poröse Schicht mit einer hydrophoben Lipidschicht beschichtet werden kann.

Aus der Druckschrift US 2005/0221072 A1 sind weitere herkömmliche implantierbare Medizinvorrichtungen bekannt, wobei zur Vermeidung von bakteriellen Entzündungen und zur Verbesserung einer osteoblastären Funktionalität an der Oberfläche Nanofäden bzw. -drähte ausgebildet werden. Hierbei kann diese nanostrukturierte Oberfläche ferner mit einem hydrophoben Material beschichtet werden.

Schließlich ist aus der den Oberbegriff des Patentanspruchs 1 bildenden Druckschrift CN 104 645 419 A ein Gelenkimplantat zur Gewebeneubildung an einem Gelenk bekannt, wobei das Gelenkimplantat einen stiftförmigen Körper mit einem Bodenbereich, einem Deckenbereich und einem Mantelbereich aufweist, und zumindest der Deckenbereich, insbesondere der gesamte stiftförmige Körper, des Gelenkimplantats eine künstliche Trabekularstruktur aufweist, welche durch ein 3D-Druckverfahren hergestellt ist.

Der Erfindung liegt die Aufgabe zugrunde, ein Gelenkimplantat zur Gewebeneubildung am Gelenk zu schaffen, welches verbesserte Eigenschaften bei der Gewebeneubildung und insbesondere eine chondroblastäre Differenzierung von mesenchymalen Stammzellen begünstigt.

Erfindungsgemäß wird diese Aufgabe durch die Merkmale des Patentanspruchs 1 gelöst.

Durch die Realisierung einer hydrophoben Oberfläche durch eine hydrophobe Nanostrukturierung eines Untergrunds kann gezielt eine chondroblastäre Differenzierung und damit die Chondrogenese von mesenchymalen Stammzellen begünstigt werden, wodurch eine Gewebeneubildung und insbesondere eine Knorpelneubildung am Gelenk verbessert wird.

Beispielsweise kann das Material des stiftförmigen Körpers ein nicht-bioresporierbares Polymer, insbesondere PA, PEK, PEKK, PEEK und UHMWPE, oder ein bioresporierbares Polymer, z.B. PCL, aufweisen.

Alternativ kann das Material des stiftförmigen Körpers auch ein Metall, insbesondere Ti, insbesondere Reintitan Grade 1, Edelstahl, oder eine Metall-Legierung, insbesondere Ti6Al4V (auch Ti64 genannt), insbesondere CoCr, oder eine nichtbioresorbierbare Keramik, insbesondere Al2O3, ZrO2 oder eine bioresorbierbare Keramik, insbesondere Ca3(PO4)2, aufweisen, wodurch ein mechanisch hochfestes Gelenkimplantat mit verbesserten Eigenschaften zur Gebewebeneubildung realisiert werden kann.

Vorzugsweise kann die hydrophobe Oberfläche durch eine zusätzlich zur hydrophoben nanostrukturierten Oberfläche hydrophobe Beschichtung realisiert sein, wodurch sich die chondroblastäre Differenzierung besonders einfach und wirkungsvoll realisieren lässt.

Beispielsweise weist die hydrophobe chemische Beschichtung ein segmentiertes Polymer, vorzugsweise ein Polyurethan oder Polyelektrolyt oder ein hydrophob funktionalisiertes Chitosan oder Chitosanderivat auf.

Vorzugsweise kann der stiftförmige Körper ferner eine Beschichtung, insbesondere mit einem Wachstumsfaktor, aufweisen, wodurch eine Knorpeldifferenzierung sowie das Anwachsen von Gewebe und insbesondere von Knorpelmaterial am Gelenkimplantat und an der Defektstelle weiter verbessert werden.

Weiterhin kann der stiftförmige Körper zumindest in seinem Bodenbereich eine hydrophile Oberfläche zur Begünstigung einer osteoblastären Differenzierung von mesenchymalen Stammzellen aufweisen, wodurch eine Knochenbildung und somit eine Verankerung im Knochen verbessert wird.

In den weiteren Unteransprüchen sind weitere vorteilhafte Ausgestaltungen der Erfindung gekennzeichnet.

Die Erfindung wird nachstehend anhand von Ausführungsbeispielen unter Bezugnahme auf die Zeichnung näher beschrieben.

Es zeigen:
Figur 1 eine vereinfachte Schnittansicht eines Oberschenkelknochens mit erfindungsgemäßen Gelenkimplantaten;
Figuren 2A und 2B vereinfachte perspektivische Ansichten von natürlichen Trabekularstrukturen;
Figuren 3A bis 3F vereinfachte perspektivische Ansichten von künstlichen Trabekularstrukturen gemäß Ausführungsbeispielen der Erfindung;
Figur 4 eine vereinfachte perspektivische Ansicht eines Gelenkimplantats gemäß einem ersten Ausführungsbeispiel der Erfindung;
Figur 5 eine vereinfachte perspektivische Ansicht eines Gelenkimplantats gemäß einem weiteren Ausführungsbeispiel der Erfindung;
Figur 6 eine vereinfachte perspektivische Ansicht eines Gelenkimplantats gemäß einem weiteren Ausführungsbeispiel der Erfindung;
Figur 7 eine vereinfachte perspektivische Ansicht eines Gelenkimplantats gemäß einem weiteren Ausführungsbeispiel der Erfindung;
Figur 8 eine vereinfachte perspektivische Ansicht eines Gelenkimplantats gemäß einem weiteren Ausführungsbeispiel der Erfindung;
Figur 9 eine schematische Übersicht der verschiedenen Reifungsstufen von mesenchymalen Stammzellen nach Aubin 1998;
Figuren 10A und 10B ein Ausführungsbeispiel zur Herstellung einer hydrophoben chemischen Beschichtung am Beispiel von segmentierten Polyurethanen;
Figur 11A und 11B vergrößerte Ansichten von mit segmentierten PU beschichteten Ti-Substraten zur Veranschaulichung eines jeweiligen Kontaktwinkels; und

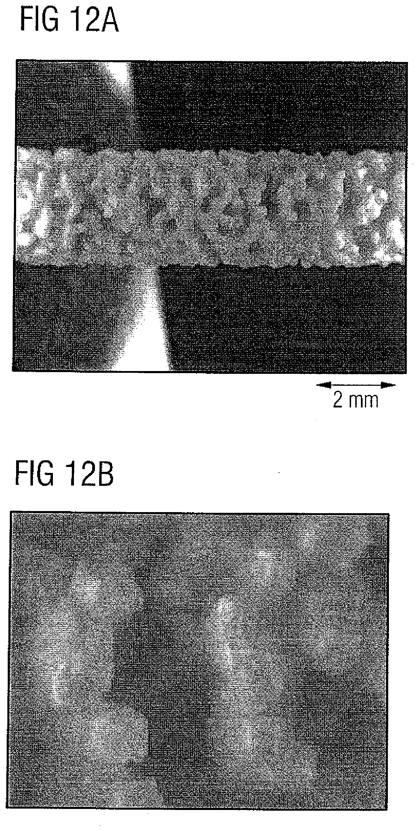

Figuren 12A und 12B eine perspektivische Ansicht sowie eine teilvergrößerte Ansicht des stiftförmigen Körpers des Gelenkimplantats gemäß einem weiteren Ausführungsbeispiel der Erfindung.

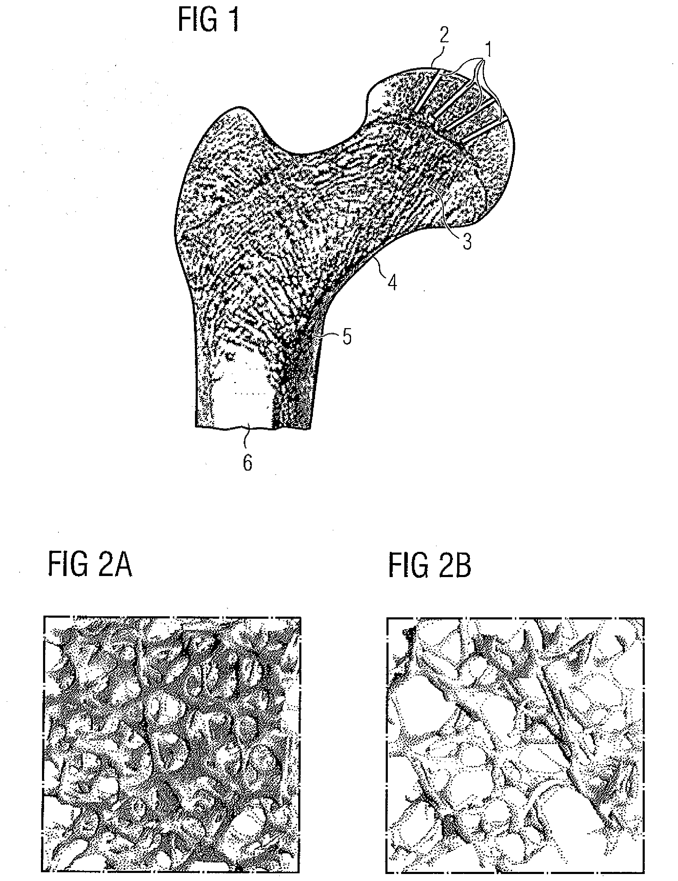

Die Figur 1 zeigt eine vereinfachte Schnittansicht eines oberen Abschnitts eines Oberschenkelknochens zur beispielhaften Veranschaulichung der Verwendung der erfindungsgemäßen Gelenkimplantate zur Gewebeneubildung an einem Oberschenkel-/Hüftgelenk. In Figur 1 sind mit Bezugszeichen 1 die erfindungsgemäßen Gelenkimplantate dargestellt, welche im Bereich des Gelenks in den Oberschenkelknochen eingebracht werden können. Hierbei kann beispielsweise an beschädigten Knorpelbereichen 2 eine oder eine Vielzahl von Vertiefungen in den Knochen gebohrt, gestanzt oder anderweitig ausgebildet und anschließend ein jeweiliges Gelenkimplantat 1 in die jeweils ausgebildete Vertiefung eingebracht werden. Die jeweilige Vertiefung ist hierbei vorzugsweise derart dimensioniert, dass das eingesetzte Gelenkimplantat 1 bzw. dessen Deckenbereich nicht an der Oberfläche des Knochens bzw. Knorpels 2 herausragt, sondern mit dieser eben abschließt oder vorzugsweise der Deckenbereich des Gelenkimplantats 1 unterhalb der Oberfläche des Knochens bzw. Knorpels 2 liegt. Der aufgrund von Gelenkerkrankung (Arthrose) geschädigte Gelenkknorpel 2 kann mit den erfindungsgemäßen Gelenkimplantaten 1 zumindest teilweise regeneriert werden, da an den Enden bzw. Deckenbereichen der eingebrachten Gelenkimplantate 1 eine Neubildung von Gewebe und insbesondere von Gelenkknorpel erfolgt.

Gemäß Figur 1 weist der Knochen eine den Knochen bedeckende Knochenhaut 4 auf, wobei in den Endbereichen 3 des Knochens eine natürliche Trabekularstruktur vorliegt, welche als sogenannte Spongiosa bezeichnet wird. Ferner besteht der Knochen in seinem mittleren Bereich aus einer relativ festen Knochenrinde 5 und in seinem Inneren aus einer Knochenmarkhöhle 6.

Durch die Verwendung einer künstlichen Trabekularstruktur zumindest an einer Oberfläche des Gelenkimplantats 1 können verbesserte Eigenschaften bei der Gewebeneubildung am Gelenk erzielt werden, was nachfolgend im Einzelnen erläutert wird.

Die Figuren 2A und 2B zeigen vereinfachte perspektivische Ansichten von natürlichen Trabekularstrukturen, wie sie beispielsweise im spongiösen Knochenbereich 3 des menschlichen Oberschenkelknochens vorliegen. Hierbei ist gemäß Figur 2A bei einem jungen, gesunden Menschen der spongiöse Knochenbereich 3 mit einer sehr feinen und dichten natürlichen Trabekularstruktur durchsetzt, während gemäß Figur 2B ein älterer und insbesondere ein an z.B. Osteoporose erkrankter Mensch oftmals eine stark veränderte natürliche Trabekularstruktur mit nur wenigen und sehr dünnen Knochenbälkchen (Trabekel) im spongiösen Knochenbereich 3 besitzt.

Das Gelenkimplantat 1 weist einen stiftförmigen Körper auf, der zumindest an seiner Oberfläche oder auch vollständig eine künstliche Trabekularstruktur aufweist. Durch die künstliche und zumindest teilweise offene bzw. für Flüssigkeiten durchlässige Trabekularstruktur des Gelenkimplantats 1 ist beispielsweise ein schnelles Besiedeln der trabekularen Oberfläche und insbesondere des Deckenbereichs des stiftförmigen Körpers mit knorpelbildenden Zellen wie z.B. Chondroblasten ermöglicht, was ein signifikant beschleunigtes und zugleich dauerhaftes Überwachsen zur Folge hat und ferner einen hochwertigen regenerativen Knorpel ermöglicht.

Das Gelenkimplantat 1 wird mit seiner künstlichen Trabekularstruktur durch geeignete 3D-Druckverfahren so aufgebaut und im Hinblick auf die bio-medizinische Anwendung so mikrostrukturiert, dass durch eine definierte Innen- und Außen-Struktur und Rauheit eine optimale Verankerung mit der natürlichen Trabekularstruktur in der Vertiefung (Bohrung, Stanzung etc.) im spongiösen Knochenbereich 3 erfolgt. Eine vertikale, laterale und ggf. rotatorische Beweglichkeit des Gelenkimplantats 1 in der Vertiefung kann dadurch ausgeschlossen werden.

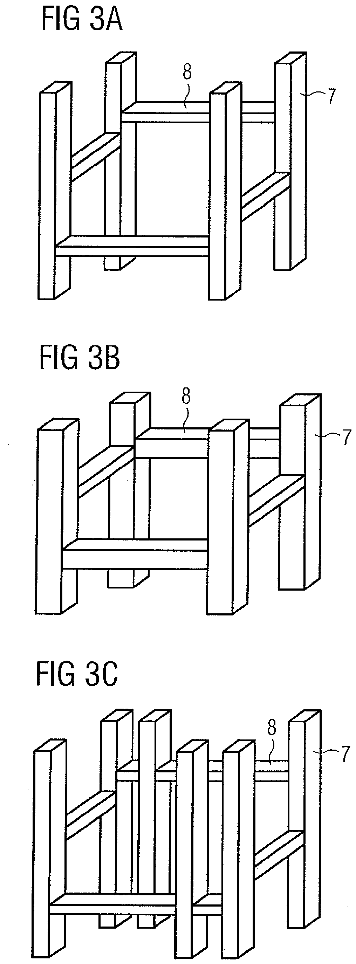

Die Figuren 3A bis 3F zeigen vereinfachte perspektivische Ansichten von künstlichen Trabekularstrukturen gemäß Ausführungsbeispielen der vorliegenden Erfindung. Die künstliche Trabekularstruktur weist hierbei eine Vielzahl von balkenförmigen oder plattenförmigen Elementen (Trabekel) auf, welche miteinander verbunden eine 3-dimensionale Mikroarchitektur ergeben.

Hierbei sollten die durch vorzugsweise 3D-Drucktechnik hergestellten, biomimetischen künstlichen Trabekel bestimmte Parameter nicht unterschreiten bzw. überschreiten.

Nachfolgend werden die maßgeblichen Parameter der beispielhaften künstlichen Trabekularstruktur näher definiert.

Die sogenannte *"*Mean Trabecular Thickness*"* (Tb.Th) definiert die durchschnittliche Trabekeldicke der jeweiligen Trabekel bzw. balkenförmigen Elemente. Da beispielsweise gemäß Figur 3A die jeweiligen Trabekel unterschiedlich ausgeformt sein können, stellt Tb.Th den Durchschnitt der lokalen Dicken aller künstlichen Trabekel dar. Die lokale Dicke ergibt sich z.B. bei rechteckförmigen Trabekeln aus der Trabekel-Diagonale und bei kreisförmigen Trabekeln aus dem Trabekel-Durchmesser. Die Figur 3B zeigt schematisch die Auswirkungen auf die künstliche Mikrostruktur bei einer Zunahme der durchschnittlichen Trabekeldicke Tb.Th. Vorzugsweise liegt die durchschnittliche Trabekeldicke Tb.Th für die künstliche Trabekularstruktur in einem Bereich von 100 bis 500 µm und insbesondere von 150 bis 400 µm.

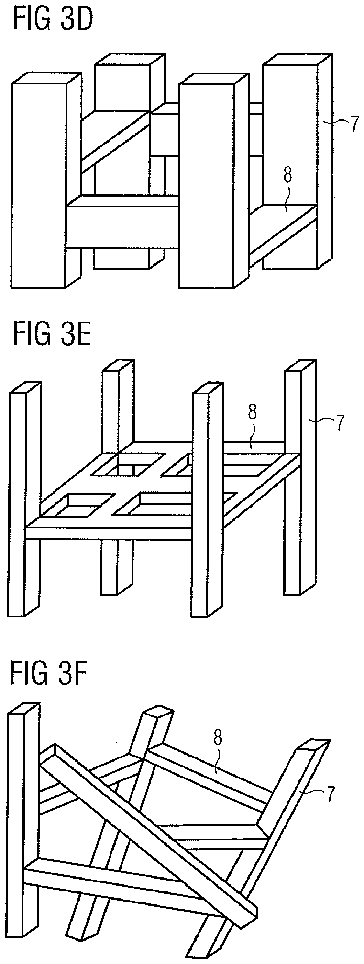

Die sogenannte *"*Mean Trabecular Separation*"* (Tb.Sp) definiert den durchschnittlichen Trabekelabstand analog zur durchschnittlichen Trabekeldicke Tb.Th. Eine Abnahme von Tb.Sp kann aus der Änderung verschiedener anderer Parameter resultieren, z.B. Zunahme von Tb.Th (Figur 3B), Zunahme von Tb.N (Figur 3C) oder Abnahme des *"*Structure Model Index*"* SMI (Figur 3D). Die Einheit für den durchschnittlichen Trabekelabstand Tb.Sp ist um und liegt für die beispielhafte künstliche Trabekularstruktur vorzugsweise in einem Bereich zwischen 100 µm und 900pm und insbesondere zwischen 200 µm und 600 µm.

Die sogenannte *"*Trabecular Number*"* (Tb.N) ist definiert als Umkehrfunktion der mittleren Distanz zwischen den Achsen der Platten und/oder Balken und gibt die Trabekelanzahl pro mm an. Die Figur 3C zeigt beispielsweise eine Zunahme von Tb.N im Vergleich zu Figur 3A. Vorzugsweise liegt die Trabekelanzahl Tb.N für die künstliche Trabekularstruktur in einem Bereich von 1 bis 6 pro mm, insbesondere in einem Bereich von 1,6 bis 5,2 pro mm.

Der sogenannte *"*Structure Model Index*"* (SMI) ist ein weiterer beschreibender Parameter der künstlichen Trabekularstruktur, bei dem es sich beispielsweise um ein aus Platten- und Stabähnlichen Elementen aufgebautes Netzwerk handeln kann. In der Realität nimmt das trabekuläre Netzwerk jedoch nicht die eine oder andere Form an, sondern es besteht ein fließender Übergang. Mit zunehmendem Alter geht z.B. ein eher Plattenähnliches Netzwerk in ein eher Stabähnliches über. Ausgehend von dieser Erkenntnis wurde daher der sogenannte *"*Structure Model Index*"* (SMI) eingeführt, der es ermöglicht, die Struktur in Bezug auf die Anzahl der Platten und Stäbe zu quantifizieren. Für ein ideales Plattenmodell liegt der SMI bei einem Wert von 0 (d.h. reine Plattenstruktur), für ein ideales Stabmodell bei einem Wert von 3. Der SMI beschreibt also die relative Zusammensetzung der künstlichen Trabekularstruktur aus Platten und Stäben. Figur 3D zeigt schematisch eine Abnahme von SMI. Der SMI ist dimensionslos und liegt für die vorliegende Erfindung beispielsweise bei 0,2 bis 2,0, vorzugsweise bei 0,25 bis 1,8.

Die sogenannte *"*Connectivity-Density (Conn.D) ist ein Maß für die Vernetzung des trabekulären Geflechts. Konnektivität ist die maximale Anzahl von Verbindungen, die innerhalb des Netzwerkes z.B. durch Mikrofrakturen unterbrochen werden können, ohne das Netz als Ganzes in zwei nicht mehr miteinander verbundene Teile zu unterbrechen. Die Figur 3E zeigt schematisch eine Zunahme der Vernetzungsdichte Conn.D. Vorzugsweise liegt die Vernetzungsdichte Conn.D für die beispielhafte künstliche Trabekularstruktur in einem Bereich von 1/mm³ bis 60/mm³, insbesondere von 1,5/mm3 bis 45/mm3.

Beim geometrischen Grad der Anisotropie (DA) handelt es sich um einen Parameter zur Quantifizierung der räumlichen Asymmetrie. Je höher DA, desto mehr liegt eine Orientierung der künstlichen Trabekularstruktur in einer bestimmten Richtung vor. Die Figur 3F zeigt schematisch eine Abnahme von DA. DA ist wie der Parameter SMI dimensionslos. Ein DA von 0 gibt eine perfekt isotrope, ein DA von 1 eine perfekt anisotrope Struktur an. Ergänzend wird der Grad der Anisoptropie auch durch den sogenannten tDA (alternativer DA) mit Werten von 1, perfekt isotrop, bis unendlich, perfekt anisotrop, angegeben. Der tDA findet bei der Beschreibung der beispielhaften Struktur hier allerdings keine Anwendung. Vorzugsweise liegt der geometrische Grad der Anisotropie DA für die beispielhafte künstliche Trabekularstruktur in einem Bereich von 0,1 bis 1,0, insbesondere von 0,2 bis 0,8 und weiter bevorzugt bei 0,2 bis 0,6.

Beim sogenannten *"*Bone Volume Tissue Volume Fraction*"* (BV/TV) handelt es sich um den volumetrischen Anteil der Trabekel am Gesamtvolumen einer betrachteten Trabekularstruktur. Eine Zunahme von BV/TV kann aus der Änderung verschiedener anderer Parameter resultieren, z.B. Zunahme von Tb.Th (Figur 3B), Zunahme von Tb.N (Figur 3C) oder Abnahme von SMI (Figur 3D). Vorzugsweise liegt BV/TV für die beispielhafte Trabekularstruktur in einem Bereich von 6% bis 70%, stärker bevorzugt von 20 bis 50%.

Schließlich definiert das sogenannte *"*Marrow Star Volume*"* (MSV) eine jeweilige Trabekel-Porosität der künstlichen Trabekularstruktur. Genauer gesagt bestimmt die MSV die Größe der Hohlräume in der künstlichen Trabekularstruktur. Der arithmetische Mittelwert mMSV liegt beispielhaft vorzugsweise in einem Bereich von 0,05 mm³ bis 110 mm³, insbesondere zwischen 0,05 mm³ bis 9 mm³ und weiter bevorzugt zwischen 0,05 mm³ bis 5 mm³.

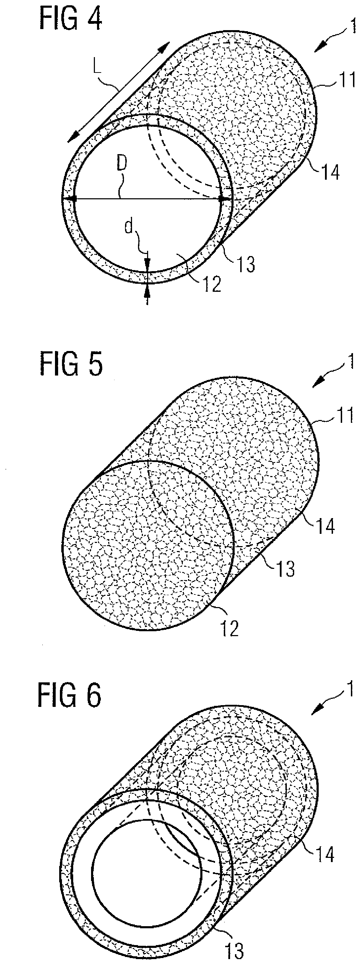

Figur 4 zeigt eine vereinfachte perspektivische Ansicht des Gelenkimplantats 1 gemäß einem ersten Ausführungsbeispiel der Erfindung. Das Gelenkimplantat 1 weist hierbei einen stiftförmigen Körper in Form eines Voll-Zylinders (flüssigkeitsdicht) auf, der einen Bodenbereich 11, einen Mantelbereich 13 und einen Deckenbereich 12 aufweist. Das Gelenkimplantat 1 kann hierbei derart in den Knochen gemäß Figur 1 eingesetzt werden, dass sein Deckenbereich 12 im Bereich des Knorpels 2 vorzugsweise leicht vertieft im Knochen angeordnet ist. Somit kann der Deckenbereich 12 des Gelenkimplantats 1 als Wachstumsbereich für das neu auszubildende Gewebe bzw. den neu auszubildenden Gelenkknorpel 2 wirken. Der Bodenbereich 11 sowie der untere Teil des Gelenkimplantats befinden sich vorzugsweise teilweise oder vollständig im spongiösen Knochenbereich 3.

Gemäß Figur 4 kann zumindest in einem Mantelbereich 13 die vorstehend beschriebene künstliche Trabekularstruktur 14 ausgebildet werden. Ferner weist der Deckenbereich 12 die künstliche Trabekularstruktur auf. Durch die offene und für Körperflüssigkeiten durchlässige Trabekularstruktur des Gelenkimplantats 1 wird beispielsweise ein schnelles Besiedeln der trabekularen Oberfläche mit Zellen wie Chondroblasten ermöglicht, was ein signifikant beschleunigtes Überwachsen zur Folge hat. Ferner ermöglicht das erfindungsgemäße Gelenkimplantat je nach Struktur und Beschichtung das Wachstum eines regenerativen Faserknorpels bis hin zu einem hochwertigen, hyalinen regenerativen Knorpels insbesondere am Deckenbereich 12.

Vorzugsweise hat der stiftförmige Körper des Gelenkimplantats 1 eine Länge L von mindestens 0,6 cm und maximal 1,2 cm für die patellare und Applikation in Kleingelenken wie, z.B. dem Hand- oder Sprunggelenk und mindestens 0,8 cm und maximal 2,2 cm, insbesondere 1,0 cm bis 1,6 cm und weiter bevorzugt 1,25 cm, für die jeweils proximale und distale tibiale und femurale Applikation im Knie- und Hüftgelenk. Dadurch kann ein Optimum an Zugänglichkeit und Einwachsen von mesenchymalen Stammzellen ermöglicht werden. Der stiftförmige Körper des Gelenkimplantats 1 kann ferner einen Durchmesser D von mindestens 2 mm und maximal 6 mm, vorzugsweise 3 mm, aufweisen, wodurch ein Optimum an lateraler, der Synovia zugewandten Oberfläche zur Ausbildung von Ersatzknorpelgewebe erzielbar ist. Eine Dicke d der künstlichen Trabekularstruktur 14 (z.B. des Mantelbereiches 13) beträgt vorzugsweise 0,5 bis 2,0 mm, weiter bevorzugt 0,5 bis 1,5 mm.

Durch eine definierte, repetitive, an die natürliche Trabekularstruktur angelehnte Netzstruktur der künstlichen Trabekularstruktur oder eine Kanalstruktur mit entsprechend angepasster Form des Gelenkimplantats 1 oder eine Kombination aus Netzstruktur und Kanalstruktur kann ein optimales Einwachsen von körpereigenem Gewebe in das Grenzvolumen zwischen Gelenkimplantat 1 und Vertiefung bzw. Bohrkanal, insbesondere in das Innenvolumen des Mantelbereichs 13 des Gelenkimplantats 1 und oberhalb des zur Synovia (Gelenkspalt) zeigenden Endes des Gelenkimplantats 1 ermöglicht werden.

Vorzugsweise werden die Gelenkimplantate 1 als mikrostrukturierte Stifte auf Basis von medizinisch zugelassenen, bioinerten und biokompatiblen, 3D-Druck-fähigen Materialien wie beispielsweise nicht-bioresporbierbaren Polymeren, insbesondere Polyamid (PA), Polyetherketone, insbesondere PEK [Polyetherketon], PEKK [Poly(etherketonketon)], PEEK [Polyetheretherketon], Polyethylen (PE), insbesondere UHMWPE [ultra high molecular weight polyethylene], oder z.B. bioresorbierbaren Polymeren, insbesondere PCL [Poly-ε-Caprolacton] ausgebildet.

Alternativ können auch, vorzugsweise 3D-Druck geeignete, Metalle und Metall-Legierungen, insbesondere Titan (Reintitan Grade 1), insbesondere Ti64 (Ti6Al4V), Ti64 ELI und TiCP, Edelstahl, insbesondere 316L, und Cobaltchrom-Legierungen, insbesondere CoCr als Materialien für die Gelenkimplantate 1 und insbesondere für deren künstliche Trabekularstrukturen verwendet werden.

Ferner können auch nicht-bioresporbierbare, vorzugsweise 3D-Druck geeignete, Keramiken, insbesondere AluminiumoxidKeramik [Al2O3], und Zirkondioxid-Keramik [ZrO2], oder bioresporbierbare Keramiken, insbesondere Calciumphosphat-Keramik [Ca3(PO4)2] als Materialien für die Gelenkimplantate 1 verwendet werden.

Grundsätzlich können auch weitere medizinisch zugelassene, bioinerte und biokompatible, sowie insbesondere 3D-Druckfähige Materialien für die Gelenkimplantate 1 und insbesondere für die künstlichen Trabekularstrukturen 14 gemäß Figuren 3 A bis 3F verwendet werden.

Figur 5 zeigt eine vereinfachte perspektivische Ansicht des stiftförmigen Körpers des Gelenkimplantats 1 gemäß einem weiteren Ausführungsbeispiel der Erfindung, wobei gleiche Bezugszeichen gleiche oder entsprechende Elemente bezeichnen, weshalb nachfolgend auf ihre wiederholte Beschreibung verzichtet wird. Während gemäß Figur 4 der stiftförmige Körper des Gelenkimplantats 1 einen Vollzylinder aufweisen kann, wobei zumindest an dessen Manteloberfläche die erfindungsgemäße Trabekularstruktur 14 ausgebildet ist, kann gemäß Figur 5 der stiftförmige Körper des Gelenkimplantats 1 auch vollständig aus der künstlichen Trabekularstruktur 14 bestehen. Das Einschwemmen, die Proliferation und die Migration von Fibrozyten, mesenchymalen Stammzellen und Chondroblasten aus dem spongiösen Knochenbereich 3 in den Knorpeldefekt kann dadurch weiter verbessert werden.

Figur 6 zeigt eine vereinfachte perspektivische Ansicht des stiftförmigen Körpers des Gelenkimplantats 1 gemäß einem Beispiel, das nicht Teil der Erfindung darstellt, da der Deckenbereich 12 keine Trabekularstruktur aufweist, wobei gleiche Bezugszeichen gleiche oder entsprechende Elemente bezeichnen, weshalb nachfolgend auf ihre wiederholte Beschreibung verzichtet wird. Gemäß Figur 6 kann der stiftförmige Körper des Gelenkimplantats 1 vorzugsweise einen Hohlkörper, wie z.B. einen Hohl-Zylinder, aufweisen, wobei die künstliche Trabekularstruktur 14 zumindest in einem Mantelbereich 13 des HohlZylinders ausgebildet ist. Beispielsweise weist der Höhlkörper zumindest einen zusammenhängenden oder mehrere nicht zusammenhängende Hohlräume auf. Alternativ kann der Hohl-Zylinder aber auch vollständig aus der künstlichen Trabekularstruktur 14 bestehen (nicht dargestellt). Das Einschwemmen, die Proliferation und die Migration von Fibrozyten, mesenchymalen Stammzellen und Chondroblasten aus dem spongiösen Knochenbereich 3 in den Knorpeldefekt kann durch diese Vergrößerung der Interaktionsoberfläche weiter verbessert werden.

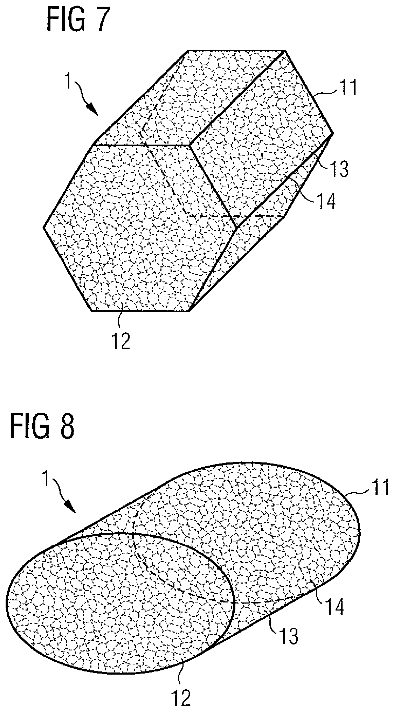

Figur 7 zeigt eine vereinfachte perspektivische Ansicht des Gelenkimplantats 1 gemäß einem weiteren Ausführungsbeispiel der Erfindung, wobei gleiche Bezugszeichen gleiche oder entsprechende Elemente bezeichnen, weshalb nachfolgend auf ihre wiederholte Beschreibung verzichtet wird. Gemäß Figur 7 kann der stiftförmige Körper des Gelenkimplantats 1 auch die Form eines Prismas oder Hohl-Prismas (nicht dargestellt) aufweisen. Durch die Makrostrukturierung, z.B. in Form eines mehreckförmigen Querschnitts, erhält man eine weiter verbesserte Verankerung des Gelenkimplantats im Knochen bzw. spongiösen Knochenbereich 3, wodurch sich die Dauerhaltbarkeit der Gelenkimplantate weiter verbessert.

Figur 8 zeigt eine vereinfachte perspektivische Ansicht des stiftförmigen Körpers des Gelenkimplantats 1 gemäß einem weiteren Ausführungsbeispiel der Erfindung, wobei gleiche Bezugszeichen gleiche oder entsprechende Elemente bezeichnen, weshalb nachfolgend auf ihre wiederholte Beschreibung verzichtet wird. Alternativ kann gemäß Figur 8 auch ein im Querschnitt ellipsenförmiger Körper oder Hohlkörper als Gelenkimplantat 1 verwendet werden, wobei wiederum eine verbesserte Verankerung und insbesondere eine verringerte rotatorische Beweglichkeit um die Längsachse zu erwarten ist, jedoch das Auftreten von unerwünschten Bruch-Segmenten an Kanten und Ecken verringert ist. Eine Dauerhaltbarkeit ist dadurch weiter erhöht.

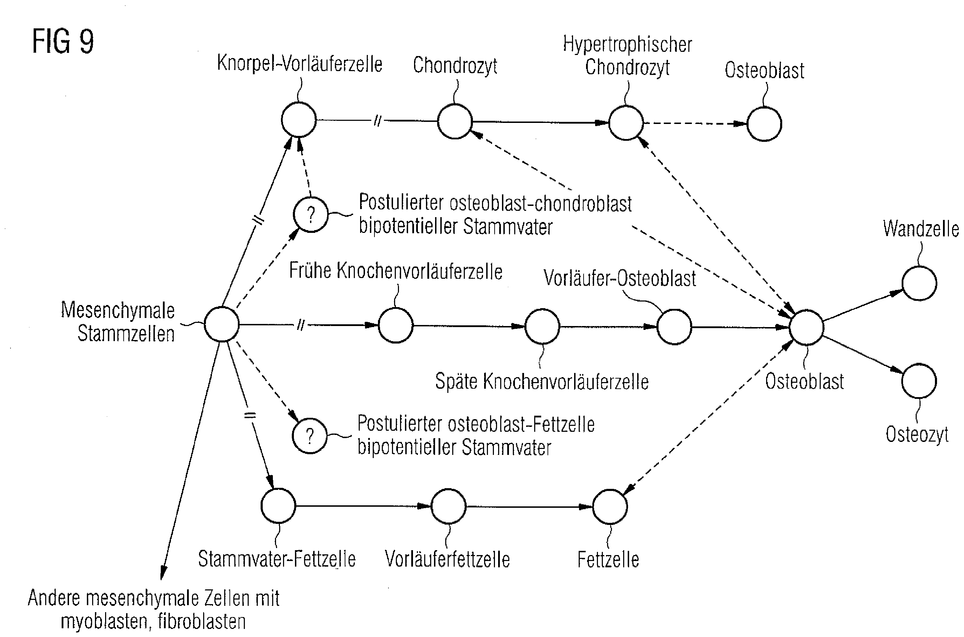

Figur 9 zeigt eine schematische Übersicht der verschiedenen Reifungsstufen von mesenchymalen Stammzellen nach Aubin 1998. Erfindungsgemäß ist es wünschenswert, dass sich mesenchymale Stammzellen (MSC) zumindest im Deckenbereich 12 des stiftförmigen Körpers des Gelenkimplantats 1 zu Chondrozyten differenzieren, um die in diesem Bereich angestrebte Knorpelneubildung zu erzeugen. Andererseits kann im unteren Teil bzw. Bodenbereich 11 des stiftförmigen Körpers des Gelenkimplantats 1 eine Differenzierung der mesenchymalen Stammzellen (MSC) hin zu Osteozyten vorteilhaft sein, um eine Knochenbildung und damit ein optimales Einwachsen des Gelenkimplantats 1 in den spongiösen Knochenbereich 3 zu begünstigen.

Überraschenderweise wurde festgestellt, dass eine derartige Differenzierung von mesenchymalen Stammzellen bereits durch die Erzeugung eines entsprechenden Untergrunds begünstigt werden kann. Genauer gesagt konnte festgestellt werden, dass eine hydrophobe Oberfläche eines Untergrunds eine chondroblastäre Differenzierung von mesenchymalen Stammzellen und somit eine Knorpelbildung begünstigt, während eine hydrophile Oberfläche eines Substrats bzw. Untergrunds eine osteoblastäre Differenzierung von mesenchymalen Stammzellen und somit eine Knochenbildung begünstigt.

Die Begriffe *"*Hydrophobie*"* bzw. *"*hydrophobe Oberfläche*"* und *"*Hydrophilie*"* bzw. *"*hydrophile Oberfläche*"* werden nachfolgend über den sogenannten Kontaktwinkel eines Wassertropfens an einer Oberfläche definiert. Hydrophobe Oberflächen weisen hierbei einen Kontaktwinkel größer oder gleich 90° auf, wobei Kontaktwinkel größer 160° superhydrophobe Oberflächen kennzeichnen. Bekanntester Vertreter für diese superhydrophoben Oberflächen ist die sogenannten *"*Lotospflanze*"*, welche durch ihre besondere Mikro- und Nanostrukturierung Kontaktwinkel von bis zu 170° aufweist. Andererseits sind hydrophile Oberflächen durch einen Kontaktwinkel kleiner 90° gekennzeichnet. Erfindungsgemäß wird nunmehr diese Differenzierungs-Eigenschaft der Stammzellen in Abhängigkeit von der Hydrophobie oder Hydrophilie einer Oberfläche dahingehend ausgenutzt, dass der stiftförmige Körper des Gelenkimplantats 1 entsprechende hydrophobe Oberflächen aufweist, die eine chondroblastäre Differenzierung der mesenchymalen Stammzellen und somit eine Knorpelausbildung begünstigen.

Hierbei kann sowohl der gesamte stiftförmige Körper eine hydrophobe Oberfläche aufweisen oder aber nur ein Teil des Körpers hydrophobe Oberflächen besitzen. Beispielsweise weist zumindest der Deckenbereich 12 eine hydrophobe Oberfläche auf, um ein Knorpelwachstum an dieser Stelle zu begünstigen. Andererseits kann der stiftförmige Körper in seinem Deckenbereich 12 und oberen Mantelbereich 13 eine hydrophobe Oberfläche aufweisen, während der Bodenbereich 11 und der untere Teil des Mantelbereichs 13 eine hydrophile Oberfläche aufweist. Dadurch kann im oberen (aus dem Knochen herausragenden) Bereich des Gelenkimplantats 1 ein Knorpelwachstum und im unteren (im Knochen befindlichen) Bereich des Gelenkimplantats ein Knochenwachstum begünstigt werden.

Erfindungsgemäß kann die hydrophobe und damit eine die chondroblastäre Differenzierung begünstigende Oberfläche auf verschiedene Art und Weise realisiert werden. Einerseits rufen geeignete Nanostrukturierungen der Oberfläche des stiftförmigen Körpers und insbesondere dessen Trabekelstrukturen eine gewünschte hydrophobe (wasserabweisende) Eigenschaft hervor. Andererseites können auch zusätzliche chemische Beschichtungen auf den nanostrukturierten stiftförmigen Körper und insbesondere dessen Trabekelstrukturen aufgebracht werden, welche die hydrophoben Eigenschaften verbessern. Weiterhin können auch Kombinationen derartiger hydrophober chemischer Beschichtungen mit physikalischen Topographie-Strukturierungen (Nanostrukturierung) zur Realisierung derartiger *"*Lotoseffekt*"*-Oberflächen mit hydrophoben Eigenschaften verwendet werden, um eine chondroblastäre Differenzierung und damit ein Knorpelwachstum zu begünstigen.

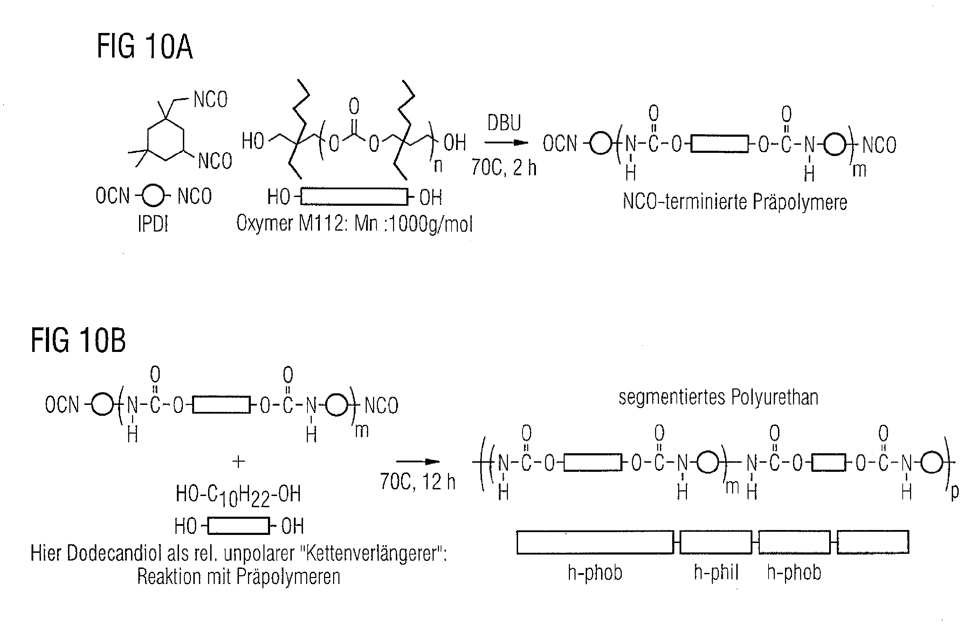

Figuren 10A und 10B zeigen ein Ausführungsbeispiel zur Herstellung einer derartigen hydrophoben Beschichtung am Beispiel von segmentierten Polyurethanen, wie sie auf ein erfindungsgemäßes Gelenkimplantat aufgebracht werden kann.

Gemäß Figur 10A wird zunächst die Herstellung von NCO-terminierten Präpolymeren veranschaulicht, wobei ein stöchiometrischer Überschuss von -NCO vorliegt. Gemäß Figur 10B werden anschließend die NCO-terminierten Präpolymere unter Verwendung von Dodecandiol als unpolaren *"*Kettenverlängerer*"* in das gewünschte segmentierte Polyurethan (segmentiertes PU) umgewandelt.

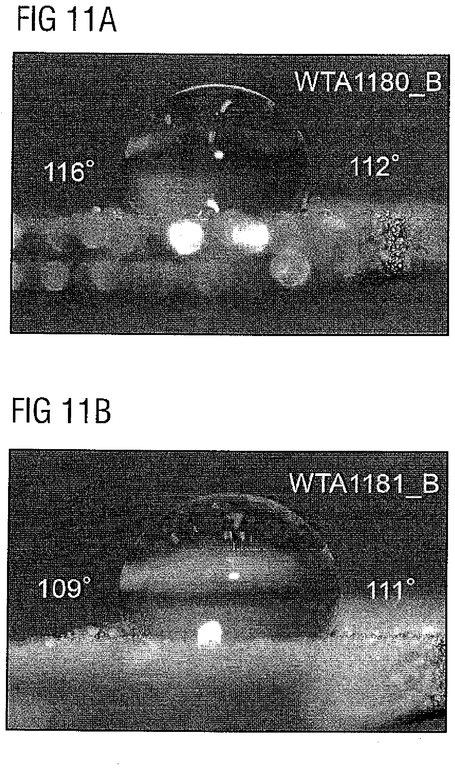

Figur 11A zeigt eine vergrößerte Ansicht eines mit einem derartigen segmentierten PU beschichteten Ti-Substrats. Während ein unbeschichtetes Ti-Substrat (nicht dargestellt) einen Kontaktwinkel von 0° aufweist, besitzt die mit segmentiertem PU beschichtete (10% PU in Toluol) Ti-Oberfläche einen Kontaktwinkel von ca. 112° bis 116°.

Figur 11B zeigt eine vergrößerte Ansicht eines mit einem segmentierten PU beschichteten Ti-Substrats, wobei eine Konzentration des segmentierten PUs bei 2% in Toluol liegt. Die mit einer derartigen segmentierten PU beschichtete Ti-Oberfläche weist nunmehr einen Kontaktwinkel von ca. 109° bis 111° auf.

Für die vorstehend beschriebene hydrophobe PU-Beschichtung wurden folgende Komponenten verwendet:
a) Aliphatische Diisocyanate: Isophoron-Diisocyanat (IPDI), Hexamethylendiisocyanat (HDI) und Dicyclohexylmethandiisocyanat (Hydriertes MDI, HMDI)
b) Polyole: Polycarbonat-Diole (Hydrolysebeständigkeit), wie z.B. Desmophen C2200, Desmophen XP2586, sowie Kohlenwasserstoff-Diole auf der Basis von Naturkautschuk und hydriertem Naturkautschuk
c) Kettenverlängerer: aliphatische Diole wie Hexandiol, Decandiol und eventiuell längere Diole wegen der Hydrophobie

Ferner kann als hydrophobe chemische Beschichtung auch ein Polyelektrolyt-Komplex auf PU-Basis verwendet werden. Hierbei werden die gleichen Komponenten wie oben verwendet, wobei zusätzlich sulfonierte Diole oder ammoniumgruppenhaltige Diole als Kettenverlängerer verwendet werden, um ionische Gruppen für die Bildung der Elektrolytkomplexe einzuführen.

Die Komplexbildung erfolgt dann nach Beschichtung (vorzugsweise Tauchbeschichtung) durch Eintauchen in eine verdünnte Lösung mit einem Tensid (kationisch oder anionisch, je nach dem, welche ionischen Gruppen im Polymer vorhanden sind). Die ionischen Wechselwirkungen zwischen dem Polyelektrolyten und dem Tensid führen zu einer festen Bindung, vor allem, wenn das Tensid hydrophob ist und daher ohnehin in wässriger Umgebung keine Tendenz hat, in Lösung zu gehen.

Ferner kann als hydrophobe chemische Beschichtung auch ein Polyelektrolyt-Komplex auf Acrylat-Basis verwendet werden, wobei eine erste Schicht aus Polyelektrolyten wie Polyacrylsäure bzw. acryl- oder methacrylsäurehaltigen Copolymeren, evtl. auch mit einigen Phosphorsäuregruppen (Comonomer Vinylphosphonsäure) zur Haftung auf der Oberfläche aufgebracht wird und anschließend eine zweite Schicht wie oben aufgebracht wird (Beschichtung aus einer Tensid-Lösung, abgestimmt auf die ionischen Gruppen des Polyelektrolyten).

Vorzugsweise werden folgende drei Typen von hydrophoben Beschichtungsmaterialien verwendet:
Polyurethan vernetzt
Polyurethan unvernetzt
Polyelektrolyt-Komplexe
welche nachfolgende Charakteristika aufweisen:
   Polyurethan vernetzt:
   Polyurethane unterscheiden sich von den meisten anderen Polymeren und Kunststoffen dadurch, daß sie durch ein *"*Baukastensystem*"* aus vielen unterschiedlichen Komponenten (Diisocyanaten, Polyisocyanaten, Polyolen, Kettenverlängerern, Weichsegmenten etc.) zusammengesetzt werden. Dabei erfolgt der eigentliche Aufbau (chemische Synthese der Polymermoleküle) typischerweise erst während der Verarbeitung, so daß der Anwender bzw. Hersteller von Bauteilen auf der Basis von Polyurethanen die endgültigen Eigenschaften ganz nach seinen Anforderungen zusammenstellen kann. Nahezu alle anderen Kunststoffe werden dagegen mit festen Eigenschaftsprofilen vom Rohstoffhersteller (Chemische Industrie) hergestellt und ausgeliefert, so daß der Anwender bzw. Hersteller von Bauteilen nur verhältnismäßig geringen Einfluß auf das Eigenschaftsprofil hat. Daher können Polyurethane eine sehr gute Ausgangsbasis für Spezialentwicklungen wie der Beschichtung des erfindungsgemäßen Implantatkörpers 1 darstellen.

Polyurethane werden seit langem als biokompatible Werkstoffe eingesetzt, beispielsweise als inerte, nicht-abbaubare Beschichtung von Herzschrittmachern, oder auch als biokompatible, abbaubare Trägermaterialien (Scaffolds) für Tissue Engineering bzw. Regenerative Medizin. Die Eigenschaften (z.B. Hydrophobie/Hydrophilie, Abbaubarkeit/Langzeitstabilität, Festigkeit, Steifigkeit, Porosität etc.) werden dabei durch die Kombination der Komponenten nach Bedarf eingestellt. Vernetzte Polyurethane werden in verdünnter Lösung in Gegenwart des zu beschichtenden Substrats hergestellt. Dabei können die Komponenten so gewählt werden, daß während der Vernetzung gleichzeitig auch eine chemische Anbindung an die Oberfläche des zu beschichtenden Substrats erfolgt. Diese Materialien haften oft ohne Haftvermittler oder ähnliche Zwischenschichten hervorragend, besonders auf hydrophilen Oberflächen. Dabei können die Komponenten der Polyurethane so gewählt werden, daß die entstehenden Schichten selbst hydrophob sind.

Als Komponenten sind für die Biokompatibilität aliphatische Di- und Polyisocyanate geeignet, für die Langzeitstabilität Weichsegmente und Polyole auf Polycarbonat-, Silicon- oder Polybutadien-Basis, und für die Hydrophobie langekttige Diole, eventuell ebenfalls auf Silicon- oder Polybutadien-Basis als Kettenverlängerer.

Der Hauptnachteil ist die problematische Kontrolle der Schichtdicke bei der Beschichtung. Die Konzentration ist der einzige unabhängige Parameter, dessen Variation die Schichtdicke beeinflussen kann. Zwar wirkt sich auch die Zusammensetzung und die Reaktionszeit auf die Schichtdicke aus, allerdings beeinflusst die Zusammensetzung auch alle anderen Eigenschaften, und die Reaktionszeit kann nicht beliebig festgesetzt werden, denn für die Biokompatibilität ist die vollständige Umsetzung der Isocyanatgruppen erforderlich, so daß die Beschichtungszeit nicht beliebig verkürzt werden kann.

### Polyurethane, unvernetzt:

Unvernetzte Polyurethane werden getrennt vom Beschichtungsvorgang hergestellt und anschließend aus einer verdünnten Lösung in einem Tauchvorgang aufgebracht. Die Einstellung der Eigenschaften bietet die gleichen Möglichkeiten wie bei den vernetzten Polyurethanen, da fast alle Komponenten in beiden Fällen eingesetzt werden können.

Der Vorteil der unvernetzten Polyurethane liegt darin, daß Synthese und Beschichtung getrennt voneinander ablaufen, so daß bessere Möglichkeiten zur Steuerung der Schichtdicke bestehen. Die Konzentration, die Einwirkzeit beim Tauchvorgang, und vor allem die Anzahl der Tauchvorgänge (mit jeweils Trocknung dazwischen) bestimmen die Dicke der aufgebrachten Schicht.

Der Nachteil liegt darin, daß eine chemische Anbindung der Schicht an das Substrat entweder eine Haftvermittler-Schicht erfordert, oder spezielle Komponenten im Polyurethan, die mit der Oberfläche reagieren können. Unter Umständen ist die Haftung dieser Schichten daher weniger dauerhaft, oder der Beschichtungsvorgang ist aufwändiger, da vor der eigentlichen Beschichtung zunächst eine Haftvermittlerschicht aufgebracht werden muß. Da dies aber voraussichtlich ebenfalls als einfache Tauchbeschichtung möglich ist, ist der zusätzliche Aufwand begrenzt.

### Polyelektrolytkomplexe:

Polyelektrolytkomplexe machen sich elektrostatische Wechselwirkungen zwischen positiv und negativ geladenen Ionen und Oberflächen zu nutze. Jedes Material weist in Wasser eine bestimmte Oberflächenladung auf (Zeta-Potential), die - je nach der chemischen Struktur - positiv oder negativ ist. Diese Oberflächenladung besitzen auch neutrale Partikel bzw. Oberflächen. Polyelektrolyte, deren Ladungen entlang der Polymerkette dieser Oberflächenladung entgegengesetzt geladen sind, haften auf der Oberfläche sehr fest. Im Allgemeinen können sie nicht mehr entfernt werden, da jede Polymerkette je nach Kettenlänge mit dutzenden oder hunderten Gruppen gleichzeitig haftet und so selbst dann in Position gehalten wird, wenn einige dieser Gruppen durch äußere Einwirkungen abgelöst werden. Auf diese Polyelektrolyte können dann entweder gegensinnig geladene Polyelektrolyte abgeschieden werden, so daß eine auf molekularer Basis genaue Einstellung der Schichtdicke durch abwechselnde Anscheidung (layer-by-layer-Technik) möglich ist. Oder es werden niedermolekulare Ionen, z.B. Tenside bzw. Seifen abgeschieden, deren eines Ende eine dem Polyelektrolyten gegensinnige Ladung trägt um die Haftung sicherzustellen, und deren anderes Ende hydrophob ist. Im Idealfall können so Schichten hergestellt werden, die nach Außen hin eine dichte Schicht an z.B. Methylgruppen aufweisen, womit nahezu die Oberflächenspannung von Fluorpolymeren (PTFE, Teflon) erreicht werden kann.

Die Vorteile dieser Materialien liegen in der üblicherweise hervorragenden Haftung in wässrigen oder nicht-wässrigen Systemen, in der exakten Steuerbarkeit der Schichtdicke, und in der relativ gut kontrollierbaren, sehr ausgeprägten Hydrophobie.

Nachteilig ist die Abscheidung in nahezu monomolekularen Schichten, die bei größeren Schichtdicken ein große Zahl von Tauchvorgängen in abwechselnden Polyelektrolytbädern erfordert. Da allerdings keine Trockenschritte dazwischen erforderlich sind, ist der Aufwand vertretbar.

Ferner können als Ausgangsmaterial für den stiftförmigen Körper des Gelenkimplantats 1 derartige 3D-druckfähige Materialien verwendet werden, die bereits per se (also ohne zusätzliche Mikro- und/oder Nanostrukturierung und/oder chemische Beschichtung eine hydrophobe Oberfläche aufweisen. Beispielsweise zeigt die unbehandelte Oberfläche eines Zirkondioxid-Keramik-Substrats bereits hydrophobe Eigenschaften.

Alternativ oder zusätzlich zur vorstehend beschriebenen hydrophoben chemischen Beschichtung oder Materialauswahl können ferner mikro- und nanostrukturierte Oberflächen am stiftförmigen Körper des Gelenkimplantats 1 erzeugt werden, wie sie als selbstreinigende Oberflächen auch unter dem Begriff Lotus-Effect^{®} bekannt sind. Derartige mikro- und nanostrukturierte Oberflächen sind beispielsweise aus der Druckschrift WO 96/04123 A bekannt.

Darüber hinaus können die künstlichen Trabekularstrukturen 14 zum Zweck der verbesserten Knorpelzelldifferenzierung und des verbesserten Anwachsens von Knorpelmaterial eine zusätzliche Wachstumsbeschichtung bzw. einen Wachstumsfaktor aufweisen. Vorzugsweise kann die künstliche Trabekularstruktur 14 mit spezifischem, humanem und human-homologem Wachstumsfaktor FGF [Fibroblast Growth Factor] insbesondere FGF-1, FGF-2 und FGF-10 bis FGF-22 und dort insbesondere FGF-18 beschichtet werden. Alternativ kann die künstliche Trabekularstruktur 14 mit spezifischem, humanem und human-homologem Wachstumsfaktor SDF [Stromal Cell-Derived Factor] insbesondere SDF-1 beschichtet werden. Ferner kann spezifischer, humaner und human-homologer Wachstumsfaktor IGF-1 [Insulin-like Growth Factor 1], humanes PDGF [Platelet-Derived Growth Factor], spezifischer, humaner und human-homologer Wachstumsfaktor TGF-β1 und TGF-β3 [Transforming Growth Factors beta 1 und beta 3], oder spezifischer, humaner und human-homologer BMP-2 und BMP-7 [Bone Morphogenetic Protein-2 und Protein-7] auf die künstliche Trabekularstruktur 14 aufgebracht werden. Weitere Möglichkeiten der Beschichtung umfassen: spezifisches, humanes und humanhomologes OP-1 [Osteogenic Protein-1], humanes PRP [Plateletrich Plasma]sowie speziell für Beschichtungen geeignetes bio-inertes Polyamid. Selbstverständlich sind auch Kombinationen der vorstehend beschriebenen Beschichtungen möglich. Vorzugsweise kann der Wachstumsfaktor als letzte Schicht aufgebracht werden.

Erfindungsgemäß kann durch entsprechende Auswahl von geeigneten bioinerten und biokompatiblen Materialien mit idealer Anpassung der geometrischen und chemisch/biochemischen Oberflächenstruktur (künstliche Trabekularstruktur) die Differenzierung von mesenchymalen Stammzellen zu Chondroblasten oder Osteoblasten gezielt gesteuert werden. Dadurch kann insbesondere die Knorpelstruktur auf der der Synovia zugewandten Seite (Deckenbereich 12) der Gelenkimplantate 1 durch die vorstehend beschriebenen hydrophoben Oberflächenstrukturen und Beschichtungen sowie mit den die Knorpelbildung anregenden Wachstumsfaktoren verbessert werden. Weiterhin kann auf der der Synovia abgewandten Seite (Bodenbereich 11) der Gelenkimplantate durch hydrophile Oberflächenstrukturen und Beschichtungen die Knochenbildung sowie die Knochenstruktur im spongiösen Knochenbereich verbessert werden. Dadurch erhält man nahezu physiologische Adäquatheit, da die Gelenkanatomie und die natürliche Knochenstabilität nicht oder nur unbedeutend beeinflusst werden wie z.B. bei Implantation einer Endoprothese. Die Verträglichkeit und Wirksamkeit der kurativen Therapie mittels vorstehend beschriebener Gelenkimplantate ist dadurch wesentlich verbessert.

Durch die Kombination der vorstehend beschriebenen biokompatiblen, bio-inerten, 3D-druckfähigen Materialien, der spezifisch geeigneten bio-medizinischen Geometrien (Krümmung, Nanostruktur, Mikrostruktur und Makrostruktur) und der wachstumsfördernden Beschichtungen erhält man ein neuartiges Gelenkimplantat, welches die Quantität und Qualität und damit die Belastbarkeit und Beständigkeit von Ersatzknorpelgewebe weiter optimieren kann und wesentlich zur kurativen Behandlung von Gelenkerkrankungen (Arthrose) beitragen kann.

Die Erfindung wurde vorstehend anhand von bevorzugten Ausführungsbeispielen beschrieben. Insbesondere kann auch Kohlenstoff als Material für die Gelenkimplantate und insbesondere für die künstlichen Trabekularstrukturen verwendet werden.

Obwohl die Erfindung vorstehend im Zusammenhang mit der Verwendung in menschlichen Hüft- und Kniegelenken beschrieben wurde, ist sie nicht darauf beschränkt und umfasst insbesondere auch menschliche Klein- sowie Kleinstgelenke (z.B. Fußsowie Finger-Gelenke) und Gelenke von Tieren.

### Bezugszeichenliste

- 1: Gelenkimplantat
- 2: Gelenkknorpel
- 3: spongiöser Knochenbereich
- 4: Knochenhaut
- 5: Knochenrinde
- 6: Knochenmarkhöhle
- 7, 8: Trabekel
- 11: Bodenbereich
- 12: Deckenbereich
- 13: Mantelbereich
- 14: künstliche Trabekularstruktur
- D: Durchmesser des Gelenkimplantats
- L: Länge des Gelenkimplantats
- d: Dicke der künstlichen Trabekularstruktur

## Patentansprüche

1. Gelenkimplantat zur Gewebeneubildung an einem Gelenk, wobei das Gelenkimplantat (1) einen stiftförmigen Körper mit einem Bodenbereich (11), einem Deckenbereich (12) und einem Mantelbereich (13) aufweist, und
zumindest der Deckenbereich (12), insbesondere der gesamte stiftförmige Körper, des Gelenkimplantats (1) eine künstliche Trabekularstruktur (14) aufweist, welche durch ein 3D-Druckverfahren hergestellt ist,
**dadurch gekennzeichnet, dass**
eine hydrophobe Oberfläche durch eine hydrophobe Nanostrukturierung eines Untergrunds realisiert ist.

2. Gelenkimplantat nach Patentanspruch 1, **dadurch gekennzeichnet, dass** der stiftförmige Körper des Gelenkimplantats (1) einen Hohlkörper mit zumindest einem oder mehreren Hohlräumen darstellt.

3. Gelenkimplantat nach einem der Patentansprüche 1 oder 2, **dadurch gekennzeichnet, dass** der stiftförmige Körper des Gelenkimplantats (1) die Form eines Zylinders, elipsenförmigen Körpers oder Prismas aufweist.

4. Gelenkimplantat nach einem der Patentansprüche 1 bis 3, **dadurch gekennzeichnet, dass** das Material des stiftförmigen Körpers ein Polymer, insbesondere PA, PEK, PEKK, PEEK, UHMWPE oder PCL, aufweist.

5. Gelenkimplantat nach einem der Patentansprüche 1 bis 4, **dadurch gekennzeichnet, dass** das Material des stiftförmigen Körpers ein Metall, insbesondere Ti, Edelstahl, oder eine Metall-Legierung, insbesondere Ti64 oder CoCr, aufweist.

6. Gelenkimplantat nach einem der Patentansprüche 1 bis 5, **dadurch gekennzeichnet, dass** das Material des stiftförmigen Körpers eine Keramik, insbesondere Al2O3, ZrO2 oder Ca3(PO4)2, aufweist.

7. Gelenkimplantat nach einem der Patentansprüche 1 bis 6, **dadurch gekennzeichnet, dass** die hydrophobe Oberfläche zusätzlich durch eine hydrophobe chemische Beschichtung realisiert ist.

8. Gelenkimplantat nach Patentanspruch 7, **dadurch gekennzeichnet, dass** die hydrophobe chemische Beschichtung ein segmentiertes Polyurethan oder Polyelektrolyt oder ein hydrophob funktionalsiertes Chitosan oder Chitosan-Derivat aufweist.

9. Gelenkimplantat nach einem der Patentansprüche 1 bis 8, **dadurch gekennzeichnet, dass** der stiftförmige Körper des Gelenkimplantats (1) eine Länge (L) von 0,6 cm bis 2,2 cm, insbesondere von 1,25 cm, aufweist.

10. Gelenkimplantat nach einem der Patentansprüche 1 bis 9, **dadurch gekennzeichnet, dass** der stiftförmige Körper des Gelenkimplantats (1) einen Durchmesser (D) von 2 mm bis 6 mm, insbesondere 3 mm, aufweist.

11. Gelenkimplantat nach einem der Patentansprüche 1 bis 10, **dadurch gekennzeichnet, dass** zumindest der Deckenbereich (12), insbesondere der gesamte stiftförmige Körper, des Gelenkimplantats (1) einen Wachstumsfaktor zur Begünstigung einer chondroblastären Differenzierung von mesenchymalen Stammzellen aufweist, insbesondere FGF-1, FGF-2, FGF-10 bis FGF-22, SDF-1, IGF-1, PDGF, TGF-β1 und TGF-β3, BMP-2 und BMP-7, OP-1, PRP oder bio-inertes Polyamid.

12. Gelenkimplantat nach einem der Patentansprüche 1 bis 11, **dadurch gekennzeichnet, dass** zumindest der Bodenbereich (11) des Gelenkimplantats (1) eine hydrophile Oberfläche aufweist.

## Claims

1. Joint implant for new tissue formation at a joint, the joint implant (1) comprising a pinlike body having a bottom region (11), a top region (12) and a shell region (13), and
at least the top region (12), more particularly the entire pinlike body, of the joint implant (1) has an artificial trabecular structure (14) produced by means of a 3D printing process,
**characterized in that**
a hydrophobic surface is realized through hydrophobic nanostructuring of a substrate.

2. Joint implant according to Claim 1, **characterized in that**
the pinlike body of the joint implant (1) constitutes a hollow body having at least one or more cavities.

3. Joint implant according to either of Claims 1 and 2, **characterized in that** the pinlike body of the joint implant (1) has the form of a cylinder, ellipsoid or prism.

4. Joint implant according to any of Claims 1 to 3, **characterized in that** the material of the pinlike body comprises a polymer, more particularly PA, PEK, PEKK, PEEK, UHMWPE or PCL.

5. Joint implant according to any of Claims 1 to 4, **characterized in that** the material of the pinlike body comprises a metal, more particularly Ti, stainless steel, or a metal alloy, more particularly Ti64 or CoCr.

6. Joint implant according to any of Claims 1 to 5, **characterized in that** the material of the pinlike body comprises a ceramic, more particularly Al2O3, ZrO2 or Ca3(PO4)2.

7. Joint implant according to any of Claims 1 to 6, **characterized in that** the hydrophobic surface is realized additionally through a hydrophobic chemical coating.

8. Joint implant according to Claim 7, **characterized in that** the hydrophobic chemical coating comprises a segmented polyurethane or polyelectrolyte or a hydrophobically functionalized chitosan or chitosan derivative.

9. Joint implant according to any of Claims 1 to 8, **characterized in that** the pinlike body of the joint implant (1) has a length (L) of 0.6 cm to 2.2 cm, more particularly of 1.25 cm.

10. Joint implant according to any of Claims 1 to 9, **characterized in that** the pinlike body of the joint implant (1) has a diameter (D) of 2 mm to 6 mm, more particularly 3 mm.

11. Joint implant according to any of Claims 1 to 10, **characterized in that** at least the top region (12), more particularly the entire pinlike body, of the joint implant (1) has a growth factor for promoting chondroblastic differentiation of mesenchymal stem cells, more particularly FGF-1, FGF-2, FGF-10 to FGF-22, SDF-1, IGF-1, PDGF, TGF-β1 and TGF-β3, BMP-2 and BMP-7, OP-1, PRP or bioinert polyamide.

12. Joint implant according to any of Claims 1 to 11, **characterized in that** at least the bottom region (11) of the joint implant (1) has a hydrophilic surface.

## Revendications

1. Implant articulaire pour la nouvelle formation de tissu au niveau d'une articulation, l'implant articulaire (1) comportant un corps en forme de broche présentant une zone de fond (11), une zone supérieure (12) et une zone d'enveloppe (13) et
au moins la zone supérieure (12), en particulier le corps total en forme de broche, de l'implant articulaire (1) présentant une structure trabéculaire (14), qui est fabriquée par un procédé d'impression 3D,
**caractérisé en ce qu'**une surface hydrophile est réalisée par une nanostructuration hydrophobe d'un substrat.

2. Implant articulaire selon la revendication 1, **caractérisé en ce que** le corps en forme de broche de l'implant articulaire (1) représente un corps creux présentant au moins un ou plusieurs espaces creux.

3. Implant articulaire selon l'une quelconque des revendications 1 ou 2, **caractérisé en ce que** le corps en forme de broche de l'implant articulaire (1) présente la forme d'un cylindre, d'un corps ellipsoïde ou d'un prisme.

4. Implant articulaire selon l'une quelconque des revendications 1 à 3, **caractérisé en ce que** le matériau du corps en forme de broche présente un polymère, en particulier PA, PEK, PEKK, PEEK, UHMWPE ou PCL.

5. Implant articulaire selon l'une quelconque des revendications 1 à 4, **caractérisé en ce que** le matériau du corps en forme de broche présente un métal, en particulier Ti, l'acier inoxydable ou un alliage métallique, en particulier Ti64 ou CoCr.

6. Implant articulaire selon l'une quelconque des revendications 1 à 5, **caractérisé en ce que** le matériau du corps en forme de broche présente une céramique, en particulier Al₂O₃, ZrO₂ ou Ca₃(PO₄)₂.

7. Implant articulaire selon l'une quelconque des revendications 1 à 6, **caractérisé en ce que** la surface hydrophile est réalisée en plus par un revêtement chimique hydrophobe.

8. Implant articulaire selon la revendication 7, **caractérisé en ce que** le revêtement chimique hydrophobe présente un polyuréthane ou un polyélectrolyte segmenté ou un chitosane ou un dérivé de chitosane fonctionnalisé de manière hydrophobe.

9. Implant articulaire selon l'une quelconque des revendications 1 à 8, **caractérisé en ce que** le corps en forme de broche de l'implant articulaire (1) présente une longueur (L) de 0,6 cm à 2,2 cm, en particulier de 1,25 cm.

10. Implant articulaire selon l'une quelconque des revendications 1 à 9, **caractérisé en ce que** le corps en forme de broche de l'implant articulaire (1) présente un diamètre (D) de 2 mm à 6 mm, en particulier de 3 mm.

11. Implant articulaire selon l'une quelconque des revendications 1 à 10, **caractérisé**
**en ce qu'**au moins la zone supérieure (12), en particulier le corps total en forme de broche, de l'implant articulaire (1) présente un facteur de croissance pour favoriser une différenciation chondroblastique de cellules souches mésenchymateuses, en particulier FGF-1, FGF-2, FGF-10 à FGF-22, SDF-1, IGF-1, PDGF, TGF-β1 et TGF-β3, BMP-2 et BMP-7, OP-1, PRP ou un polyamide bioinerte.

12. Implant articulaire selon l'une quelconque des revendications 1 à 11, **caractérisé**
**en ce qu'**au moins la zone de fond (11) de l'implant articulaire (1) présente une surface hydrophile.
